# EUROPEAN PATENT SPECIFICATION

(11) **EP 0 981 282 B1**
(45) Date of publication and mention of the grant of the patent: **15.09.2004**
(21) Application number: 98908547.7
(22) Date of filing: 12.02.1998
(51) Int. Cl.: A23K 1/04, A23J 1/06, A23J 1/10

(54) **PROCESS FOR PREPARING A NUTRITIONAL SUPPLEMENT AND NUTRITIONAL SUPPLEMENT**
VERFAHREN ZUR HERSTELLUNG EINES NAHRUNGSERGÄNZUNGSMITTELS UND NAHRUNGSERGÄNZUNGSMITTEL
PROCEDE DE PREPARATION DE SUPPLEMENT NUTRITIONNEL ET SUPPLEMENT NUTRITIONNEL

(30) Priority: 27.02.1997 US 38863 P
(43) Date of publication of application: 01.03.2000
(73) Proprietor: Griffin Industries, Inc., Cold Spring, KY 41076-1897 (US)
(72) Inventor: GRIFFIN, Martin, W., Taylor Mill, KY 41015 (US); THOMAS, Steven, D., Falmouth, KY 41040 (US); CROWLEY, John, L., Alexandria, KY 41001 (US); WEDDINGTON, Eugene, M., Loveland, OH 45140 (US)
(74) Representative: Dunlop, Brian Kenneth Charles
(86) International application number: PCT/US1998/002854
(87) International publication number: WO 1998/037773

(56) References cited:
- WO-A-91/18520
- GB-A- 2 167 639
- US-A- 3 272 632
- US-A- 4 089 978
- US-A- 4 269 865
- US-A- 5 049 397

## Description

### Technical Field

The present invention relates generally to a process for preparing a nutritional supplement and more specifically, a nutritional supplement designed to provide the desired amino acids and essential nutrients to promote the growth of young animals.

### Background of the Invention

Food additives are substances that may be added to foods during processing in order to improve desirable chemical or physical characteristics and food quality. One of the most important forms of food additives are nutritional supplements. These typically comprise proteins, minerals and vitamins that are added to foods in order to restore nutritional values lost during food processing or to supplement the natural content of the food nutrients. The importance of the use of nutritional supplements to enhance the health and growth of farm animals has long been recognized in the field of animal husbandry.

It has also long been recognized that the production of nutritional supplements from various by-products of food rendering operations is highly desirable. Specifically, by establishing a commercial use for these by-products, the economic viability of the food rendering process is enhanced and otherwise difficult waste material handling is reduced or altogether avoided. This is a particularly important concept for the poultry rendering industry where significant amounts of waste materials are produced. Accordingly, various methods have been developed for the utilization and processing of feathers and offal as a nutritional supplement for food products. Such prior art processes are generally disclosed in, for example, US-A-3,272,632 US-A-4,269,865, US-A-5 049 397 and GB-A-2 167 639.

One of the primary drawbacks in the prior art processing of waste products such as feathers and offal into nutritional supplements has been the heat degradation of various amino acids and proteins that effectively eliminates these materials as a source of nutrition. Accordingly, a need is identified for an improved method of processing waste products such as feathers and offal so as to avoid heat degradation of important amino acids and proteins and thereby provide a supplement with enhanced nutritional values.

### Summary of the Invention

It is a primary object of the present invention to provide an economical and effective process for preparing a nutritional supplement designed to provide the desired amino acids in a highly available form to promote the growth of young animals and more particularly farm animals such as piglets.

Yet another object of the present invention is to provide a continuous process for preparing a nutritional supplement from whole blood, feathers and offal allowing far more efficient production while requiring relatively inexpensive equipment and, therefore, a minimal capital expenditure to initiate production.

Additional objects, advantages and other novel features of the invention will be set forth in part in the description that follows and in part will become apparent to those skilled in the art upon examination of the following or may be learned with the practice of the invention. The objects and advantages of the invention may be realized and obtained by means of the instrumentalities and combinations particularly pointed out in the appended claims.

To achieve the foregoing and other objects, and in accordance with the purposes of the present invention as described herein, the present method includes the step of mixing animal blood, hydrolized poultry feathers and poultry offal at respective weight ratios of approximately 4-6/2-4/1-3 depending upon the particular application of the end product.

More specifically, fresh whole blood is steam coagulated and then pumped into a decanter centrifuge to separate the liquid and solids. The solids are used in the present process. The feathers are previously heat hydrolyzed utilizing a steam treatment or other means in accordance with various methods known in the art to produce a hydrolyzed feather product of approximately 50% moisture and 50% hydrolyzed feathers that is used in the present process. In this way, the feathers undergo a partial protein breakdown so as to be more digestible with enzymes during subsequent processing. The poultry offal is used fresh.

After mixing the coagulated animal blood solids, the heat hydrolyzed feather product and the fresh poultry offal, the mixture is ground or milled in order to produce a particle size of, for example, no greater than ¼ inch in diameter. This step serves to increase the surface area of the poultry feathers and poultry offal parts in order to enhance the digestive activity of enzymes and microorganisms active in the process.

Next is the adding of a protease enzyme to the ground mixture in order to digest the mixture. Various protease enzymes and mixtures thereof may be utilized. Preferably the enzyme(s) are added at a rate of approximately one gallon per 4000 lbs of product mixture. Next is the digesting of the ground or milled mixture by the enzyme at a temperature of substantially 54-72°C for up to 35-60 minutes. This is followed by the spray drying of the digested mixture and the heating of the product to a maximum of 83°C in order to reduce the moisture content down to 5-8 weight percent and kill any pathogens present in the mixture. This is then followed by the sizing of the final product.

Advantageously, by only subjecting the mixture of feathers, animal blood and offal to enzyme hydrolysis and avoiding high processing temperatures, a more digestible food product is produced. Further, the amino acids in the food product and more particularly the blood and offal are not degraded or denatured and, therefore, remain intact for the full nutritional benefit of the feeding animal.

Still other objects of the present invention will become apparent to those skilled in this art from the following description wherein there is shown and described a preferred embodiment of this invention, simply by way of illustration of one of the modes best suited to carry out the invention. Accordingly, the drawings and descriptions will be regarded as illustrative in nature and not as restrictive.

### Brief Description of the Drawing

The accompanying drawing incorporated in and forming a part of the specification, illustrates several aspects of the present invention and together with the description serves to explain the principles of the invention. In the drawing:

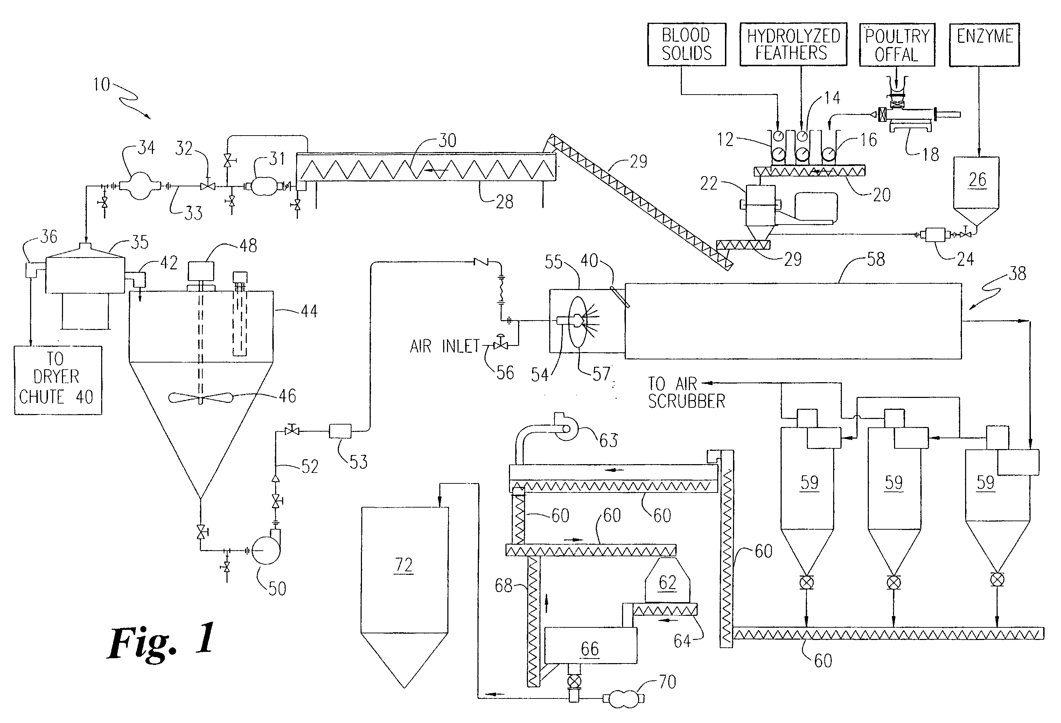

Figure 1 schematically details the process of the present invention and the equipment utilized to complete that process.

Reference will now be made in detail to the present preferred embodiment of the invention, an example of which is illustrated in the accompanying drawing.

### Detailed Description of the Invention

The process of the present invention for preparing a nutritional supplement from animal blood, hydrolyzed poultry feathers and animal offal will now be described in detail with reference to the drawing figure showing an apparatus 10 that may be utilized for this purpose. As shown, steam coagulated blood solids are delivered to a first bin 12 while hydrolyzed feathers are delivered to a second bin 14 and fresh poultry offal is delivered to a third bin 16 by means of a pump 18. Measured quantities of these ingredients are delivered from the bins 12, 14 and 16 to the screw conveyor 20 which mixes and delivers the coagulated blood solids, hydrolyzed feathers and poultry offal to a grinder 22. There the blood solids, hydrolyzed poultry feathers and poultry offal are thoroughly mixed at respective weight ratios of approximately 4-6/2-4/1-3 depending upon the particular application of the end product. As indicated above, the feathers utilized in the process have been previously heat hydrolyzed by means of a steam treatment or other means in accordance with the various methods known in the art to produce a hydrolyzed feather product of approximately 50% moisture and 50% hydrolyzed feathers. Thus, the feathers undergo a partial protein breakdown so as to be more digestible with enzymes during subsequent processing.

Protease enzyme is supplied via pump 24 from storage vessel 26 to the grinder 22. There the blood solids, hydrolyzed feather and poultry offal mixture is ground or milled in order to produce a particle size of, for example, no greater than ¼ inch in diameter. This grinding step serves to increase the surface area of the poultry feathers and poultry offal parts in order to enhance the digestive activity of the enzymes and microorganisms active in the process. Preferably, the enzymes are added at a rate of approximately one gallon per 4000 pounds of product mixture.

The thoroughly ground and mixed blood solids, hydrolyzed feathers, poultry offal and protease enzymes are delivered from the grinder 22 to a digester tank 28 by a series of screw conveyors 29. The ground or milled mixture is then digested by the enzymes in the digester tank at a temperature of substantially 54-72°C for up to 35-60 minutes. A screw conveyor 30 in the digester tank 38 insures processing of the mixture for the appropriate residence time.

Following digestion, the mixture is delivered by a positive displacement pump (pneumatic blower) 31 past valve 32 through line 33 to a masserater 34 where it is thoroughly masserated. Next, the mixture is screened using state-of-the-art screening equipment 35. Specifically, the relatively low moisture meal is separated from the mixture through the discharge outlet 36 for downstream delivery to the dryer 38 by means of the chute 40. The relatively high moisture content water phase including relatively small digested hydrolyzed feather and poultry offal particulates is delivered through the outlet 42 into the mixing tank 44. The mixing tank 44 includes an agitator 46 driven by a motor 48 which insures that the particulates remain in suspension. A pump 50 delivers the water phase and suspended digested particulates past various valves in line 52 through a flow meter 53 to a spray nozzle 54 in the burner section 55 of dryer 38. Simultaneously, compressed air is delivered through the inlet 56 (at a pressure of substantially 60-85 and more preferably 75-80 psig) and directed to the nozzle 54 so that the water phase is sprayed within the burner ring 57 for rapid drying in a manner described in greater detail in co-pending U.S. Patent Application S.N. 08/841,230 filed April 29, 1997, entitled "Method and Apparatus for Drying and Processing Raw Food Material", the disclosure of which is fully incorporated herein by reference.

Following flash drying in the burner section 55, the sprayed material mixes with the meal delivered through the chute 40 in the rotary section 58 of the dryer 38. As a result of the combined drying action the moisture content of the mixture is reduced down to approximately 5-8 weight percent.

Now the dried feed mixture is air delivered to a series of cyclones 59 which function to recover the mixture which is then delivered by a series of screw conveyors 60 to a mill 62. During delivery, a cooler fan 63 provides a flow of cooling air in order to maintain the mixture at a lower temperature. In the mill 62 the feed mixture is milled to the desired final product size. The final product is then delivered by screw conveyor 64 to a screen 66 for sizing. The resulting product may be recycled from the screen 66 by means of the screw conveyors 68, 60 back to the mill 62 to rescreen. In most instances, the feed product is delivered by operation of the pneumatic blower 70 to a storage vessel 72 where it is held for packaging or further processing.

In summary, numerous benefits result from employing the concepts of the present invention. By only subjecting the mixture of hydrolyzed feathers, blood solids and offal to enzyme hydrolysis and avoiding high processing temperatures above approximately 83°C, a more digestible food product is produced. Further, the amino acids in the food product and more particularly the blood and offal are not degraded or denatured and therefore, remain intact for the full nutritional benefit of the feeding animal.

In the foregoing description of a preferred embodiment of the invention an obvious modification includes the use of offal, other than poultry offal in the described process.

## Claims

1. A process for preparing a nutritional supplement providing desired amino acids to promote the growth of young animals, **characterised in that** said process comprises:
mixing animal blood, hydrolyzed poultry feathers and animal offal to create a mixture;
adding protease enzyme to said mixture;
digesting said mixture with said protease enzyme; and
drying said digested mixture in order to reduce the moisture content thereof down to 5-8 weight percent and kill any pathogens present in said mixture.

2. The process set forth in claim 1, including coagulating said animal blood and collecting blood solids for mixing with said hydrolyzed poultry feathers and animal offal.

3. The process set forth in claim 2, wherein said animal offal is from poultry.

4. The process set forth in claim 1 or claim 2 including providing said animal blood, hydrolyzed poultry feathers and animal offal in said mixture at a respective weight ratio of substantially 4-6/2-4/1-3.

5. The process set forth in any preceding claim, further including grinding/milling said mixture prior to adding said protease enzyme in order to increase the surface area of solid particles in said mixture for enhanced enzymatic activity.

6. The process set forth in any preceding claim, further including sizing a final product following drying.

7. The process set forth in claim 6, wherein said mixture is not exposed to a temperature above substantially 60-83°C.

8. The process set forth in claim 7, wherein a temperature of approximately 83°C is not exceeded during processing.

9. A nutritional supplemental obtainable in accordance with the process of any preceding claim.

## Patentansprüche

1. Verfahren zur Herstellung einer Nahrungsmittelergänzung zur Bereitstellung angestrebter Aminosäuren zur Förderung des Wachstums von jungen Tieren,
**dadurch gekennzeichnet,**
**dass** das Verfahren die folgenden Schritte umfasst:
Vermischen von Tierblut, hydrolysierten Geflügelfedern und tierischem Abfall zur Erzeugung einer Mischung;
Zugeben von Proteaseenzym zu dieser Mischung;
Aufschießen dieser Mischung mit dem Proteaseenzym und
Trocknen der aufgeschlossenen Mischung zur Verringerung von deren Feuchtigkeitsgehalt herab auf 5 bis 8 Gew.-% und Töten von allen Pathogenen, die in dieser Mischung vorhanden sind.

2. Verfahren gemäß Anspruch1 einschließlich des Koagulierens des Tierblutes und des Sammelns der Blutfeststoffe zum Vermischen mit den hydrolysierten Geflügelfedern und dem Tierabfall.

3. Verfahren gemäß Anspruch 2, wobei der Tierabfall von Geflügel stammt.

4. Verfahren gemäß Anspruch 1 oder Anspruch 2 einschließlich der Bereitstellung des Tierblutes, der hydrolysierten Geflügelfedern und des Tierabfalls in der Mischung in einem entsprechenden Gewichtsverhältnis von im wesentlichen 4-6/2-4/1-3.

5. Verfahren gemäß einem der vorangehenden Ansprüche, darüber hinaus einschließlich des Zerkleinerns/Mahlens dieser Mischung vor der Beigabe des Proteaseenzyms, um die Oberfläche der Feststoffpartikel innerhalb der Mischung zu erhöhen für eine verstärkte enzymatische Aktivität.

6. Verfahren gemäß einem der vorangehenden Ansprüche, darüber hinaus einschließlich der Klassifizierung eines Endproduktes nach dem Trocknen.

7. Verfahren gemäß Anspruch 6, wobei die Mischung keiner Temperatur oberhalb im wesentlichen 60 - 83°C ausgesetzt wird.

8. Verfahren gemäß Anspruch 7, wobei eine Temperatur von etwa 83°C während der Bearbeitung nicht überschritten wird.

9. Nahrungsergänzungsmittel, erhältlich gemäß dem Verfahren eines der vorangehenden Ansprüche.

## Revendications

1. Procédé de préparation d'un supplément nutritionnel fournissant des acides aminés désirés pour favoriser la croissance de jeunes animaux, **caractérisé par le fait que** ledit procédé comprend les opérations consistant à :
- mélanger du sang animal, des plumes de volailles hydrolysées et des déchets d'abattage d'animaux pour créer un mélange ;
- ajouter une enzyme protéase audit mélange ;
- faire digérer ledit mélange par ladite enzyme protéase ; et
- sécher ledit mélange digéré de façon à réduire la teneur en humidité de celui-ci en descendant jusqu'à 5-8 pour cent en poids et tuer tous pathogènes présents dans ledit mélange.

2. Procédé selon la revendication 1, comprenant la coagulation dudit sang animal et la collecte des matières solides du sang en vue du mélange avec lesdites plumes de volailles hydrolysées et déchets d'abattage d'animaux.

3. Procédé selon la revendication 2, dans lequel lesdits déchets d'abattage d'animaux sont des déchets de volailles.

4. Procédé selon la revendication 1 ou la revendication 2, comprenant l'opération consistant à introduire ledit sang animal, lesdites plumes de volailles hydrolysées et lesdits déchets d'abattage d'animaux dans ledit mélange à un rapport en poids respectif de sensiblement 4-6/2-4/1-3.

5. Procédé selon l'une quelconque des revendications précédentes, comprenant en outre le meulage/broyage dudit mélange avant l'addition de ladite enzyme protéase de façon à augmenter l'aire de surface de particules solides dans ledit mélange pour une activité enzymatique augmentée.

6. Procédé selon l'une quelconque des revendications précédentes, comprenant en outre un dimensionnement du produit final après séchage.

7. Procédé selon la revendication 6, dans lequel ledit mélange n'est pas exposé à une température au-dessus de sensiblement 60-83°C.

8. Procédé selon la revendication 7, dans lequel une température d'approximativement 83°C n'est pas dépassée pendant le traitement.

9. Supplément nutritionnel pouvant être obtenu conformément au procédé tel que défini à l'une quelconque des revendications précédentes.
